Europäisches Patentamt

European Patent Office

Office européen des brevets

(19)

(11) **EP 1 249 347 A2**

(12) **EUROPÄISCHE PATENTANMELDUNG**

(43) Veröffentlichungstag:
**16.10.2002 Patentblatt 2002/42**

(51) Int Cl.7: **B41F 33/00**

(21) Anmeldenummer: **02405268.0**

(22) Anmeldetag: **05.04.2002**

| | |
|---|---|
| (84) Benannte Vertragsstaaten:<br>**AT BE CH CY DE DK ES FI FR GB GR IE IT LI LU MC NL PT SE TR**<br>Benannte Erstreckungsstaaten:<br>**AL LT LV MK RO SI** | (71) Anmelder: **Maschinenfabrik Wifag**<br>**3001 Bern (CH)** |
| (30) Priorität: **12.04.2001 DE 10118604** | (72) Erfinder: **Langsch, Robert**<br>**3042 Ortschschwaben (CH)** |

(54) **Verfahren und Vorrichtung zur Beurteilung der Qualität einer Druckfarbe**

(57) Die Erfindung bezieht sich auf ein Verfahren zur Beurteilung der Qualität einer Druckfarbe, wobei eine bestimmte Menge der Druckfarbe gerührt wird; eine beim Rühren aufzuwendende Kraft bzw. Moment gemessen wird und eine definierte Menge Feuchtmittel zu einem bestimmten Zeitpunkt zur Farbe zugegeben wird sowie eine Vorrichtung zur Beurteilung der Qualität der Druckfarbe mit einem Behälter für die Druckfarbe; einer Rührvorrichtung, mit welcher eine in dem Behälter eingefüllte Farbe gerührt werden kann; einer Messvorrichtung zur Messung der von der Rührvorrichtung aufzubringenden Kraft bzw. dem aufzubringenden Moment; einer Feuchtmittelzufuhr und einer Steuerung, welche die Feuchtmittelzufuhr so ansteuert, dass eine vorgegebene Menge des Feuchtmittels zu einem definierten Zeitpunkt in den Behälter eingegeben wird.

Figur 2

EP 1 249 347 A2

**Beschreibung**

Die vorliegende Erfindung bezieht sich auf Verfahren und eine Vorrichtung zur Beurteilung der Qualität bzw. zur Analyse einer Druckfarbe, insbesondere zur Beurteilung der Eignung einer untersuchten Druckfarbe für den Druck, insbesondere den Offset-Druck.

Neben den Druckmaterialien haben die verwendete Farbe sowie das Verhalten der Farbe in Verbindung mit Wasser einen großen Einfluss auf das Druckresultat und auch auf die Stabilität des über längere Zeit und unter unterschiedlichen Betriebszuständen laufenden Druckprozesses. Insbesondere für den Offset-Druck muss eine verwendete Druckfarbe in gewissem Umfang Wasser aufnehmen können.

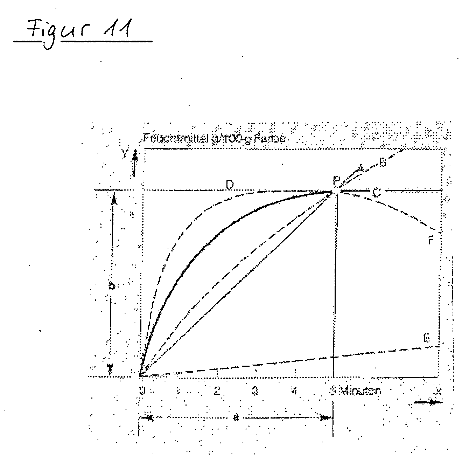

Zur Bestimmung der Wasseraufnahmefähigkeit einer Farbe ist der sogenannte Surlandtest bekannt, wonach 50 g Farbe mit 50 g Wasser verrührt werden. Nach fünf Minuten wird das von der Farbe nicht aufgenommene überschüssige Wasser abgeschüttet. Wenn die Farbe in diesen fünf Minuten 10 bis 15 g Wasser aufgenommen hat, gilt diese nach dem Surlandtest als gut, wohingegen die Farbe als für den Offset-Druck ungeeignet angesehen wird, wenn sie 20 g oder mehr Wasser aufgenommen hat. Figur 11 zeigt das Feuchtmittelaufnahmeverhalten von verschiedenen verwendeten Farben A bis F über die Zeit, wobei gesehen werden kann, dass die Farben A bis D und F nach einer Dauer a eines Wasserbads von fünf Minuten in etwa die gleiche Menge b Feuchtmittel aufgenommen haben, so dass die entsprechenden Kennlinien alle durch den Gleichgewichtspunkt P verlaufen. Die Farbe E zeigt ein relativ schlechtes Wasseraufnahmeverhalten und kommt deshalb für den Offset-Druck nicht in Frage.

Weiterhin sind Handrührversuche zur Bestimmung der Sättigungsgrenze der Feuchtmittelaufnahmefähigkeit einer Farbe bekannt, wobei bei diesen Handrührversuchen nur gefühlsmäßig beurteilt wird, ob der dabei anfallende Rühraufwand groß oder klein ist. Jedoch können mit solchen Handrührversuchen keine systematischen Analysen gemacht werden. Insbesondere kann nicht vorausgesagt werden, ob eine bestimmte Druckfarbe tatsächlich im Offset-Druck verwendbar ist oder nicht.

Eine Vorrichtung zum Bestimmen des Emulgierverhaltens und der Sättigungsgrenze einer Farbe wird unter der Bezeichnung "Lithotronic Emulsification Tester" von der Firma Novocontrol verkauft. Diese Vorrichtung weist einen Behälter für eine Farbprobe auf, in welchen eine Rührvorrichtung eingreift, um eine darin eingefüllte Farbe zu rühren, wobei das beim Rühren aufzuwendende Drehmoment gemessen wird. Nach dem vom Gerätehersteller vorgeschlagenen Testablauf wird die Farbprobe zuerst bei 1200 U/min gerührt, bis das gemessene Drehmoment konstant ist. Danach wird kontinuierlich eine Wassermenge von 1,5 bis 3 g/min zugegeben. Wenn das Drehmoment zusammenbricht ist die Sättigungsgrenze der Farbe erreicht, d. h. zusätzlich zugeführtes Wasser wird von der Farbe nicht mehr aufgenommen und ermöglicht so einen leichteren Lauf der Rührvorrichtung.

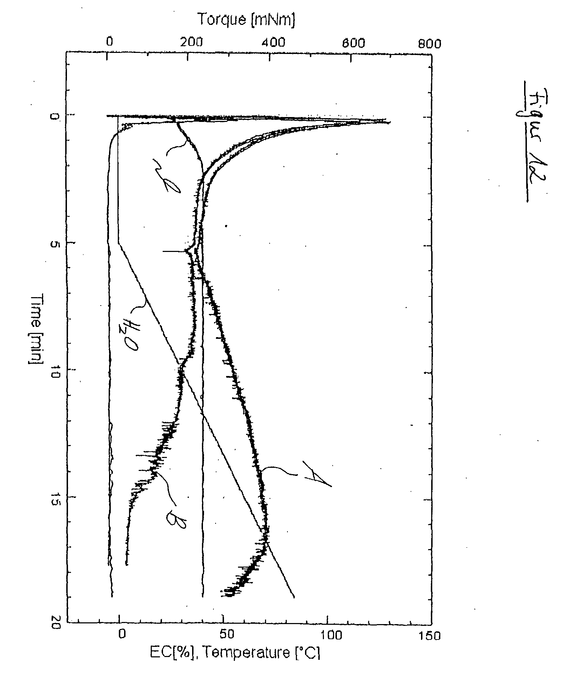

Figur 12 zeigt ein mit dieser Vorrichtung erzieltes Testergebnis, wobei zwei verschiedene Farben A und B in die Vorrichtung eingegeben wurden und während fünf Minuten kontinuierlich verrührt wurden, so dass das Drehmoment bei etwa 230 mNm und die Temperatur bei etwa 40°C konstant waren. Nach fünf Minuten wurde kontinuierlich mit der Zufuhr von Wasser begonnen. Aus Figur 12 kann gesehen werden, dass bei der Farbe A das zum Rühren aufzuwendende Drehmoment während etwas 11 Minuten nach dem Beginn der Wasserzufuhr ansteigt und dann allmählich zusammenbricht. Aus der während dieser Zeit zugeführten Wassermenge kann ermittelt werden, wie viel Wasser von der Farbprobe aufgenommen werden kann, es kann also die Sättigungsgrenze der Farbe bestimmt werden. Bei der untersuchten Farbe B bleibt das beim Rühren aufzuwendende Drehmoment während etwa vier Minuten nach Beginn der Wasserzufuhr konstant und verringert sich danach stetig. Es kann somit auch für die Farbe B die Sättigungsgrenze errechnet werden, wobei qualitativ aus dem Diagramm gesehen werden kann, dass die Wasseraufnahmefähigkeit der Farbe B niedriger liegt als diejenige der Farbe A. Als weiterer Parameter ist aus dem Diagramm von Figur 12 abzulesen, dass die Farbe A eine steigende Nassviskosität aufweist, d. h. dass die Viskosität bei Zufuhr von Wasser zunächst ansteigt, während die Farbe B eine sinkende Nassviskosität zeigt, also bei Wasserzufuhr kontinuierlich dünnflüssiger wird. Obwohl dieses Farbuntersuchungsverfahren eine gewisse Standardisierung bei der Überprüfung verschiedener Farben ermöglicht, sind die ermittelten Parameter, nämlich das Nassviskositätsverhalten und die Sättigungsgrenze, im Wesentlichen ungeeignet um Aussagen darüber zu treffen, ob eine untersuchte Farbe im Druckprozess gute oder schlechte Ergebnisse liefert.

Es ist eine Aufgabe der vorliegenden Erfindung Verfahren und eine Vorrichtung vorzuschlagen, mit welchen Parameter zu Beurteilung der Qualität einer Farbe ermitteln werden können. Insbesondere sollen erfindungsgemäß Verfahren und eine Vorrichtung vorgeschlagen werden, welche eine Aussage ermöglichen, ob eine Farbe für einen Druckprozess eher geeignet oder eher ungeeignet sein wird bzw. worin das bei der Verwendung dieser Farbe auftretende Problem besteht.

Diese Aufgabe wird gelöst durch die Gegenstände der unabhängigen Ansprüche. Vorteilhafte Ausführungsformen ergeben sich aus den abhängigen Ansprüchen.

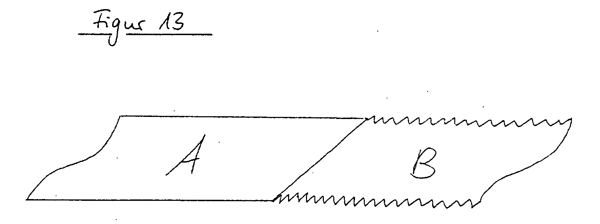

Der Erfindung liegt folgende Erkenntnis zugrunde, welche unter Bezugnahme auf Figur 13 nachfolgend beschrieben wird. Figur 13 zeigt schematisch die Oberfläche einer Druckplatte, wobei nebeneinanderliegend ein farbfreundlicher glatter Bereich A und ein wasserfreundlicher rauer oder poröser Bereich B gezeigt sind. Während des Druckprozesses wird auf die Druckplatte vor dem eigentlichen Druckvorgang zunächst Wasser aufgebracht, welches auf dem glatten Bereich A als dünner Wasserfilm aufliegt und vom rauen oder porösen Bereich B in vergleichsweise größerem Umfang aufgenommen wird. Anschließend wird eine Farbe auf die Druckplatte aufgebracht. Die Farbe nimmt den an den farbfreundlichen Plattenstellen A der Druckplatte aufliegenden dünnen Wasserfilm auf und lagert sich somit an diesen Stellen an. Der raue Bereich B hat jedoch mehr Wasser aufgenommen als der glatte Bereich A und wirkt aufgrund seiner unebenen Oberflächenbeschaffenheit oder Porosität wie ein Wasserspeicher. Als Folge kann die auf den wasserfreundlichen Bereich B aufgebrachte Farbe das gesamte gespeicherte Wasser nicht aufnehmen und kann sich demzufolge nicht an dem Druckplattenbereich B anlagern und wird von der Druckplatte in einem nachfolgenden Schritt abgewaschen.

Da während des Druckvorganges nur ein bestimmter definierter Zeitraum zur Verfügung steht, während welchem eine aufgebrachte Farbe den auf der glatten Schicht A aufliegendem Wasserfilm aufgenommen haben muss, wobei die Farbe jedoch noch nicht soviel Wasser aufgenommen haben darf, dass sie sich auch auf dem wasserfreundlichen, rauen Bereich B anlagert, wurde von dem Erfinder erkannt, dass zur Beurteilung der Qualität einer Druckfarbe einerseits ermittelt werden muss, ob eine Druckfarbe Wasser nicht zu schnell aufnimmt, da sich diese Farbe sonst womöglich auch auf dem wasserfreundlichen Bereich B anlagern würde, wobei die Anfangswasseraufnahme der Druckfarbe auch nicht zu lange dauern darf, da die Druckfarbe sonst den dünnen Wasserfilm nicht aufnehmen kann und auch vom farbfreundlichen Bereich A abgewaschen werden würde.

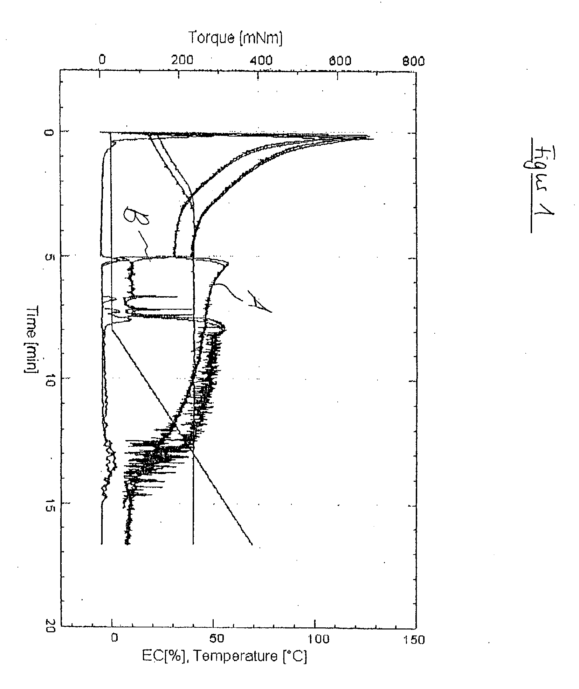

Nach dem erfindungsgemäßen Verfahren zur Beurteilung der Qualität einer Druckfarbe wird eine bestimmte Menge der Farbe von z. B. 25g zunächst gerührt. Unter Rühren im Sinne der Erfindung wird sowohl ein Rühren mittels eines geeigneten Drehelements, aber auch jedes andere Verfahren verstanden, mit welchem eine Durchmischung einer Flüssigkeit erzielt werden kann. Wird ein Rührelement verwendet, so kann dieses zum Beispiel auch geeignet durch die Farbe bewegt werden ohne dabei eine Drehbewegung auszuführen, um so die Farbe zu rühren bzw. zu durchmischen. Es ist auch möglich einen Farbbehälter mit oder auch ohne darin befindlichem Rührelement geeignet zu bewegen, zum Beispiel zu drehen oder wiederholt seitlich etwas zu kippen, um die Farbe zu rühren. Dabei wird die beim Rühren aufzuwendende Kraft oder ein aufzuwendendes Moment oder auch die erforderliche Leistung oder Energie gemessen. Dies kann zum Beispiel durch Messung des Drehmoments bei einem in die Farbe eingebrachten Rührelement erfolgen. Jedoch können je nach verwendetem Rührverfahren auch andere auftretende Kräfte oder Momente gemessen werden. Nachdem die Farbe eine bestimmte Zeit gerührt worden ist, bevorzugt nachdem sich das aufzuwendende Moment stabilisiert hat, wird eine definierte Menge Feuchtmittel, zum Beispiel 1 bis 50g Leitungswasser oder destilliertes Wasser, innerhalb kurzer Zeit, bevorzugt auf einmal, zur Farbe zugegeben, so dass plötzlich eine große Menge Wasser zur Verfügung steht. Dies entspricht in etwa den beim Druckprozess auftretenden Bedingungen, wobei die Farbe innerhalb sehr kurzer Zeit auf die Druckplatte aufgebracht wird und bei guter Eignung das Wasser so schnell aufnimmt, dass sie sich auf dem glatten farbfreundlichen Bereich A anlagert, wobei von der Farbe jedoch nicht so viel Wasser aufgenommen wird, dass sie sich auch auf dem wasserfreundlichen Bereich B anlagert. Nach dem erfindungsgemäßen Verfahren wird zum Beispiel anhand der gemessenen beim Rühren aufzuwendenden Kraft ermittelt, wie das Wasseraufnahmeverhalten der Farbe ist. Unmittelbar nach dem schnellen Zuführen der bestimmten Wassermenge liegen Farbe und Wasser noch im entmischten Zustand vor, so dass das Farb-/Wassergemisch leicht zu rühren ist. Wird ein Drehelement zum Rühren verwendet, so kann gesehen werden, dass unmittelbar nach der Zugabe der bestimmten Wassermenge das Drehmoment bei einem Großteil der Farben zusammenbricht, da diese Farben das Wasser nicht sofort aufnehmen können. Kann kein oder kein nennenswerter Zusammenbruch des Drehmoments gemessen werden, so ist dies ein Indiz dafür, dass die Farbe das zugegebene Wasser sehr schnell aufgenommen hat. Eine solche Farbe wäre für ein Druckverfahren ungeeignet, da sich diese Farbe mit sehr gutem und schnellem Wasseraufnahmeverhalten auch auf dem wasserfreundlichen Bereich B anlagern würde. Bricht das Drehmoment zusammen, so kann im Laufe der Zeit, gewöhnlich nach wenigen Minuten beobachtet werden, dass das Drehmoment wieder ansteigt, d. h. das zugegebene Wasser wird von der untersuchten Farbprobe allmählich aufgenommen. Aus dem Anstieg des Drehmoments kann ermittelt werden, ob die Farbe das zugegebene Wasser nach einer vorgegebenen Zeit aufnehmen kann und somit für den Druckprozess geeignet ist, oder ob das Drehmoment zu lange zusammengebrochen ist, was auf ein sehr schlechtes Wasseraufnahmeverhalten der Farbe schließen lässt. Eine solche Farbe würde sich auch nicht auf dem farbfreundlichen glatten Bereich A einer Druckplatte anlagern, da diese Farbe innerhalb der im Druckprozess zur Verfügung stehenden Zeit noch nicht einmal den auf dem farbfreundlichen glatten Bereich A aufliegenden dünnen Wasserfilm aufnehmen kann und sich somit nicht auf dem farbfreundlichen Bereich A anlagern kann, sondern von diesem abgewaschen werden würde. Nach dem erfindungsgemäßen Verfahren kann somit eine Aussage über die Geschwindigkeit der Wasseraufnahme einer Farbe getroffen werden, nachdem die Farbe plötzlich mit einer vorgegebenen Wassermenge in Kontakt kommt bzw. mit dieser gemischt wird. Es können somit zum Beispiel Diagramme erstellt werden, welche eine quantitative Aussage der beim Rühren der Farbe aufzuwendenden Kraft bzw. des aufzuwendenden Drehmoments über die Zeit darstellen, wobei insbesondere der Zeitraum nach dem schnellen Hinzugeben der bestimmten Wassermenge, dem sogenannten Wasserschock, von besonderer Bedeutung ist. Beispiele solcher Diagramme sind in den Figuren 1 bis 10 gezeigt und werden später beschrieben werden.

Obwohl in den Ausführungsbeispielen der Figuren 1 bis 10 ermittelte Vorläufe des gemessenen Drehmoments gezeigt sind, wird angemerkt, dass die Erfindung nicht auf die gezeigten Beispiele beschränkt ist. In Abhängigkeit von der untersuchten Farbmenge, der Menge des zugegebenen Wassers, dem Rührverfahren, der Temperatur oder anderen Parametern können andere Kennlinien erhalten werden, bei welchen der Kurvenverlauf einer für den Druckprozess geeigneten Farbe anders ist. Allgemein kann jedoch mit dem erfindungsgemäßen Verfahren bei einer guten Druckfarbe immer ein anderer Kurvenverlauf erhalten werden als bei einer schlechten Druckfarbe, wobei die charakteristischen Merkmale der Kurvenverläufe gegebenenfalls durch Versuche bestimmt werden können.

Bevorzugt wird die Farbe durch ein Drehelement, insbesondere einen Rührer gerührt, welcher in einen Behälter eingebracht wird, in welchem sich die Farbe befindet. Dabei kann das Rührelement zum Beispiel im oberen Bereich der eingefüllten Farbprobe platziert sein, d. h. dass zum Beispiel ein Rührelement eine Rührbewegung nur im oberen Bereich der eingefüllten Farbmasse durchführt und nicht bis zum Boden des Farbbehälters hineinragt, so dass bei plötzlichem Zugeben von Wasser das Rührelement eine Rührbewegung überwiegend im Kontaktbereich zwischen Wasser und Farbe durchführt, bevor der Vermischungsprozess stattfindet.

Vorteilhaft wird das Rühren der Farbprobe ohne und/oder mit zugegebenem Feuchtmittel kontinuierlich durchgeführt, also zum Beispiel bei einer konstanten Drehzahl. Alternativ kann auch zuerst mit einer ersten zum Beispiel höheren Rate gerührt werden bis zum Beispiel ein aufzuwendendes Drehmoment in etwa konstant und die Farbe gleichmäßig verflüssigt ist, wobei dann mit einer zweiten zum Beispiel niedrigeren Rate gerührt wird, um nach der Feuchtmittelzugabe eine bessere zeitliche Auflösung der gemessenen Kraft-, Moment- oder Energiekurve zu erhalten.

Besonders bevorzugt wird die Farbprobe zunächst bei einer ersten Drehzahl während eines ersten Zeitraumes gerührt, zum Beispiel während zwei Minuten bei 1200 U/min, wonach die Drehzahl für das weitere Verfahren geändert wird, insbesondere abgesenkt wird, zum Beispiel auf 300 U/min, um eine bessere zeitliche Auflösung der Kennlinien zu erhalten. Wird nach Zugabe des Feuchtmittels mit einer hohen Drehzahl gerührt, so erfolgt aufgrund der besseren Durchmischung eine schnellere Aufnahme des Feuchtmittels durch die Farbprobe. Wird jedoch die Farbprobe zunächst bei einer höheren Drehzahl gerührt, um einen definierten Ausgangszustand zu erhalten, um zum Beispiel die Farbe aufgrund des tixotropen Verhaltens der Farbprobe zu verflüssigen, so kann die Wasseraufnahmegeschwindigkeit durch eine Absenkung der Drehzahl verlängert werden, so dass eine bessere zeitliche Auflösung erreicht wird. Bei einem durchgeführten Test wurde zum Beispiel ermittelt, dass der Unterschied der Wasseraufnahmegeschwindigkeit zweier Farben bei 1200 U/min ca. 20 % betrug, während sich die Wasseraufnahmegeschwindigkeit bei einer Drehzahl von 300 U/min um den Faktor 10 unterschied, d. h. bei langsameren Drehzahlen bzw. Rührvorgängen können genauere Aussagen mit dem erfindungsgemäßen Verfahren getroffen werden.

Bevorzugt ist die Temperatur der Farbprobe einstellbar bzw. regelbar, zum Beispiel durch ein Thermostat bzw. ein mit einem Temperatursensor gekoppeltes geregeltes Heizelement, um Messungen immer bei einem vorgegebenen, vorzugsweise konstanten Temperaturbereich durchzuführen. Insbesondere wird bevorzugt die Messung im Temperaturbereich von 20°C bis 60°C, vorteilhaft von 30°C bis 50°C und insbesondere im Bereich von 40°C durchgeführt.

Es ist vorteilhaft die gesamte definierte Menge des Feuchtmittels der Farbprobe erst dann zuzugeben, wenn die beim Rühren aufzuwendende Kraft oder ein Drehmoment und/oder die Temperatur konstant ist. Bei praktischen Versuchen hat sich gezeigt, dass nach ca. drei bis fünf Minuten ausreichend konstante Werte für die Temperatur und die beim Rühren aufzuwendende Kraft bzw. ein Drehmoment erreicht wurden.

Vorteilhaft wird nach dem einmaligen Zugeben einer definierten Menge des Feuchtmittels nach einem vorgegebenen Zeitraum weiteres Feuchtmittel mit einer kontinuierlichen Rate zugegeben, so dass die Sättigungsgrenze der Farbe ermittelt werden kann, d. h. es wird ermittelt wann das Drehmoment zusammenbricht, so dass hieraus die maximale Feuchtmittelaufnahmefähigkeit der Farbe berechnet werden kann, d. h. welche Menge Feuchtmittel pro Farbmenge aufgenommen werden kann. Bei kontinuierlicher Feuchtmittelzugabe kann, wie oben beschrieben, ermittelt werden, ob die Nassviskosität einer Farbe steigt oder fällt, d. h. ob die Zähflüssigkeit einer Farbe bei langsamer Zugabe von Feuchtmittel ansteigt oder abfällt.

Gemäß einem anderen Aspekt der Erfindung kann der Feuchtmittel- bzw. Wassergehalt einer Farbprobe, welche zum Beispiel einem laufenden Druckprozess entnommen wurde, bestimmt werden, um Aussagen über den Verlauf der Wasseraufnahme während des Druckprozesses zu erhalten. Es wird also zu Beginn eines Druckverfahrens eine Frischfarbe verwendet, welche während des Druckprozesses an verschiedenen Stellen mit Feuchtmittel in Kontakt kommen kann und dabei unterschiedliche Mengen an Feuchtigkeit aufnimmt. Wird nun an einer bestimmten Stelle des Druckverfahrens eine Farbprobe zum Beispiel von einer Druckplatte entnommen, so kann durch Bestimmung des Feuchtmittelgehalts dieser Farbprobe ermittelt werden, ob diese Farbprobe für das Druckverfahren geeignet ist oder zum Beispiel aufgrund zu schneller oder zu langsamer Feuchtmittelaufnahme zu unbefriedigenden Druckergebnissen geführt hat oder führen wird. Erfindungsgemäß wird zunächst die Sättigungsgrenze der Frischfarbe ermittelt, indem zum Beispiel wie oben beschrieben kontinuierlich Wasser zu einer gerührten Farbe zugegeben wird, bis diese kein Wasser mehr aufnehmen kann und die beim Rühren aufzuwendende Kraft aufgrund der Verdünnung der gesättigten Farbe durch überschüssiges Feuchtmittel abnimmt, also zum Beispiel ein Rührdrehmoment einbricht. Die so bestimmte Sättigungsgrenze der Frischfarbe dient als Bezugsgröße für die spätere Berechnung. Der zu untersuchenden Farbprobe wird erfindungsgemäß Feuchtmittel bei andauerndem Rühren zugegeben, bis die Sättigungsgrenze der zu untersuchenden Farbprobe erreicht wird. Dies kann zum Beispiel wiederum wie oben beschrieben durch die Messung der bei einem Rührvorgang aufzuwendenden Kraft erfolgen. Aus diesen beiden ermittelten Werten, d. h. der Sättigungsgrenze der Frischfarbe und der Menge des zur Farbprobe zugegebenen Feuchtmittels kann errechnet werden, wie viel Feuchtmittel bzw. Wasser schon von der Farbprobe vor der weiteren Zugabe des Feuchtmittels aufgenommen wurde. Wird zum Beispiel eine Farbprobe von 30 g untersucht, bei welcher nach anfänglichem Rühren mit zum Beispiel 1200 U/min während einer Zeit von zwei Minuten die Drehzahl auf 300 U/min abgesenkt wird, wobei nach drei Minuten Rühren mit 300 U/min begonnen wird kontinuierlich mit 2,5 g/min Feuchtmittel zuzugeben, bis das Drehmoment zusammenbricht, so kann der prozentuale Wassergehalt der Farbprobe durch folgende Formeln (1) bis (4) ermittelt werden:

$$(1) \quad \text{Masse}_{\text{(Farbprobe + Wasser)}} = 30\ g + \text{Einspritzzeit} * 2{,}5\ g/\text{min}$$

$$(2) \quad \text{Wasser}_{\text{(an Sättigungsgrenze)}} = \text{Sättigungsgrenze}_{\text{(der Frischfarbprobe)}} / 100 * \text{Masse}_{\text{(Farbprobe + Wasser)}}$$

$$(3) \quad \text{Wasser}_{\text{(der ursprünglichen Farbprobe)}} = \text{Wasser}_{\text{(an Sättigungsgrenze)}} - \text{Einspritzzeit} * 2{,}5\ g$$

$$(4) \quad \text{prozentualer Wassergehalt der Farbprobe} = (\text{Wasser}_{\text{(der ursprünglichen Farbprobe)}} / 30\ g) * 100$$

Insbesondere sollte die Sättigungsgrenze einer Farbe gemäß diesem Testablauf über 30 % liegen, um gute Druckresultate zu erhalten

Die erfindungsgemäße Vorrichtung zur Beurteilung der Qualität einer Druckfarbe weist einen Behälter auf, in welchen die zu untersuchende Farbe eingegeben werden kann. Weiterhin ist eine Rührvorrichtung vorgesehen, welche wie oben ausgeführt zum Beispiel ein in den Behälter einbringbares Rührelement oder eine Dreh- und/oder Kippvorrichtung für den Behälter mit oder ohne eingebrachtem Rührelement sein kann. Die zum Rühren der im Behälter befindlichen Farbe aufzuwendende Kraft bzw. ein aufzuwendendes Drehmoment oder eine aufzuwendende Energie oder Leistung wird durch eine Messvorrichtung gemessen und bevorzugt in Form eines Diagramms über eine Zeitachse aufgetragen. Erfindungsgemäß ist eine Feuchtmittelzufuhr und eine Steuerung für die Feuchtmittelzufuhr vorgesehen, wobei die Steuerung so ausgelegt ist, dass eine definierte Feuchtmittelmenge zu einem definierten Zeitpunkt zu der in dem Behälter befindlichen Farbe zugegeben werden kann. Anhand des von der Messvorrichtung ermittelten Kraftbzw. Drehmomentverlaufes nach Zugeben des Feuchtmittels kann dann eine qualitative Aussage über die Eignung der Farbe für den Druck, insbesondere den Offset-Druck getroffen werden.

Bevorzugt ist eine Temperaturregelvorrichtung, insbesondere ein Thermostat mit einem Temperatursensor und einer Heizvorrichtung vorgesehen, um den Behälter für die Farbe auf eine geeignete Temperatur bringen zu können, so dass Messungen immer in einem definierten Temperaturbereich vorgenommen werden können.

Vorteilhaft ist die Steuerung so ausgelegt, dass mit der erfindungsgemäßen Vorrichtung einer oder mehrere der oben beschriebenen Verfahrensschritte durchgeführt werden können, wie zum Beispiel kontinuierliche Zugabe eines Feuchtmittels nach dem Wasserschock, also der einmaligen Zugabe einer bestimmten Feuchtmittelmenge, Einstellen des Drehzahlbereiches zum anfänglichen Rühren der Farbe bei einer ersten höheren Drehzahl und zum Weiterrühren der Farbe bei einer zweiten niedrigeren Drehzahl, bei welcher das Feuchtmittel zugegeben wird, und so weiter.

Erfindungsgemäß wird auch eine Vorrichtung zur Durchführung des oben genannten Verfahrens, insbesondere in Kombination mit einer Druckmaschine, vorgeschlagen, wobei die oben beschriebene Vorrichtung zur Bestimmung der Qualität einer Druckfarbe in Kombination mit einer Druckmaschine, insbesondere einer Offset-Druckmaschine vorgesehen ist, so dass automatisch eine der Druckmaschine zugeführte Farbe vor und/oder während des Druckverfahrens untersucht bzw. analysiert wird und in Abhängigkeit von dem Untersuchungsergebnis die Einstellung der Druckmaschine automatisch geändert werden kann.

Nach einem weiteren Aspekt der Erfindung wird ein Farbaufbereitungsverfahren und eine Farbaufbereitungsvorrichtung geschaffen, welche automatisch eine zugeführte Farbe nach dem oben beschriebenen Verfahren untersuchen können und gegebenenfalls geeignete Farbzusätze zur Farbe zugeben, um diese Farbe auf den Druckprozess abzustimmen, d. h. diese Farbe für den Druckprozess geeignet zu machen. Hierzu können Farbzusatzstöffe verwendet werden, welche die Wasseraufnahmefähigkeit und/oder Wasseraufnahmegeschwindigkeit einer Farbe erhöhen oder senken. Beispielhaft wird auf die Farbzusatzstoffe Colorthix und Colorstabil der Firma Vegra verwiesen, welche die Feuchtmittelaufnahmefähigkeit einer Farbe verändern können. Demzufolge kann erfindungsgemäß auch eine an sich ungeeignete Farbe der Farbaufbereitungsvorrichtung eingegeben werden, welche in Abhängigkeit von dem Untersuchungsergebnis, also z. B. einem von einem idealen Verlauf abweichenden Drehmomentverlauf der untersuchten Farbe ein geeignetes Farbaufbereitungsmittel wie z. B. Farbpigmente, Bindemittel, Verdünnungsmittel wie z. B. Wasser, Lösungsmittel oder andere Additive wie z. B. Harz der Farbe zugibt, um diese so für das nachfolgende Druckverfahren verwendbar zu machen. Dieser Prozess kann auch selbstlernend, z. B. unter Verwendung eines neuronalen Netzes ausgestaltet sein.

Die Erfindung wird nachfolgend anhand von durchgeführten Messbeispielen veranschaulicht, wobei:

Figur 1            ein Untersuchungsergebnis zweier Farben mit unterschiedlicher Wasseraufnahmefähigkeit zeigt;
Figur 2            eine Farbanalyse eines bei einer ersten Druckmaschine verwendeten ersten Farbsatzes zeigt;
Figur 3            eine Farbanalyse eines bei der ersten Druckmaschine verwendeten zweiten Farbsatzes zeigt;
Figur 4            eine Farbanalyse für eine zweite Druckmaschine zeigt;
Figur 5 und 6    die Farbanalyse bei einer dritten Druckmaschine zeigen;
Figur 7 und 8    die Farbanalyse zweier Farbsätze bei einer Druckmaschine mit Kurzfarbwerk zeigen;
Figur 9            ein Analyseergebnis zur Voruntersuchung der Eignung eines Farbsatzes für eine Druckmaschine mit Spaltfarbwerk zeigt;
Figur 10          eine Farbanalyse bei aufgetretenem Nebeln und schlechter Förderung im Farbkasten zeigt;
Figur 11          ein Diagramm des Wassertoleranztests nach dem Surland-Model zeigt;
Figur 12          ein Diagramm eines bekannten Farbanalyseverfahrens zeigt; und
Figur 13          schematisch die Oberfläche einer Druckplatte mit farbfreundlichem Bereich A und wasserfreundlichem Bereich B zeigt.

Figur 1 zeigt prinzipiell den Verlauf des Drehmoments in mNm über die Zeit bei einer Farbprobe von 25 g, welche innerhalb der ersten fünf Minuten mit 1200 U/min gerührt wird bis das Drehmoment bei etwa 220 mNm konstant ist, wobei nach fünf Minuten 7,5 g Feuchtwasser eingespritzt wurden. Es zeigt sich, dass die Farbe A das zugeführte Feuchtwasser sehr schnell aufgenommen hat und demzufolge wahrscheinlich zu Problemen beim Offset-Druck führen wird. Bei der untersuchten Farbe B bricht das Drehmoment unmittelbar nach der Feuchtwasserzugabe zusammen auf einen Wert von etwa 80 mNm und steigt etwa zwei bis drei Minuten nach der Feuchtmittelzugabe rasch wieder an, d. h. die Farbe B zeigt ein für den Offset-Druck günstiges Wasseraufnahmeverhalten, und wird sich also auf dem in Figur 13 schematisch gezeigten farbfreundlichen Bereich A der Druckplatte schnell genug anlagern wird und wird sich nicht auf dem wasserfreundlichen Bereich B anlagern. Acht Minuten nach Beginn des Rührvorgangs, d. h. drei Minuten nach Zugabe der 7,5 g Feuchtwasser wird kontinuierlich Wasser zugegeben, bis die Sättigungsgrenze der Farben A und B erreicht ist, d. h. das Drehmoment fast vollständig zusammenbricht.

Allgemein wurde herausgefunden, dass bei der erstmaligen Zugabe eines Feuchtmittels bei guten Druckfarben das Drehmoment zusammenbrach, sich also schnell auf einen bestimmten Wert absenkte, so dass ein solcher Kurventeilverlauf als Indiz für eine gute Farbqualität spricht. Bei einer guten Druckfarbe sollte auch das Drehmoment nach kürzerer Zeit wieder ansteigen.

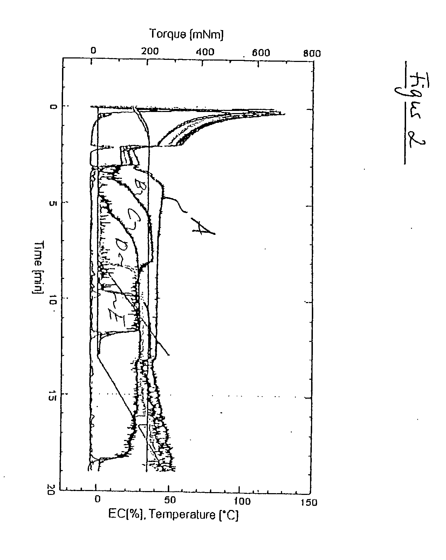

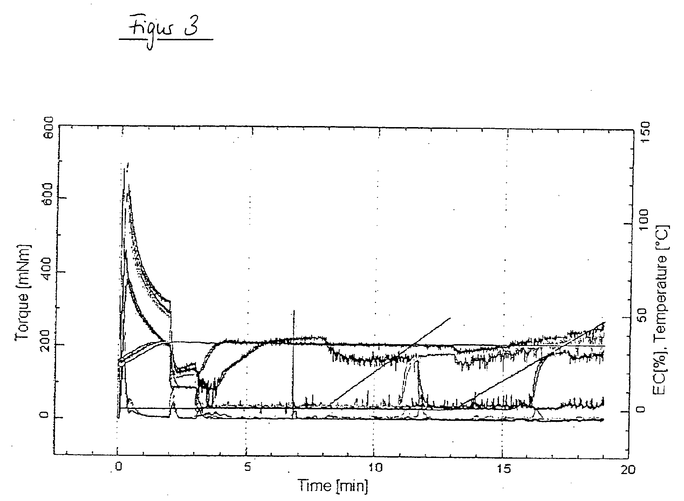

Figuren 2 und 3 zeigen Untersuchungen von verschiedenen verwendeten Farbsätzen bei der gleichen in Guangzhou installierten Druckmaschine. Aus den Diagrammen ist ersichtlich, dass nach ca. zwei Minuten die Anfangsdrehzahl abgesenkt wurde, wobei die Feuchtmittelzugabe nach ca. drei Minuten erfolgte. Die gezeigte Analysekurve für die Farbe A lässt erkennen, dass diese Farbe das Feuchtmittel zu schnell aufnimmt und somit für den Offset-Druck eher ungeeignet ist. Die Farben B und C nehmen das zugegebene Feuchtmittel nicht unmittelbar auf, wie die Farbe A, sondern nach einem gewissen Zeitraum und waren beide für den Offset-Druck geeignet. Die Farbe D hat ein zu schlechtes Feuchtmittelaufnahmeverhalten und sollte ebenso wie die Farbe E nicht für den Offset-Druck verwendet werden. Die mit dem in Figur 2 gezeigten Farbsatz betriebene Druckmaschine lief nach dem Einstellen nur kurze Zeit fehlerfrei. Anhand des in Figur 3 gezeigten Diagramms der Analyse der zuletzt verwendeten Farben konnte nachgewiesen werden, dass der Lieferant der verwendeten Druckfarben die Zusammensetzung dieser Druckfarben geändert hat, so dass diese Druckfarben Feuchtmittel entweder zu schnell oder zu langsam aufnehmen, so dass die Fehlfunktion der Druckmaschine eindeutig auf die Verschlechterung der verwendeten Druckfarben zurückzuführen war. Es sind keine Kurvenverläufe vorhanden, bei welchen das Drehmoment nach Feuchtmittelzugabe rasch einbricht und kurz darauf wieder ansteigt.

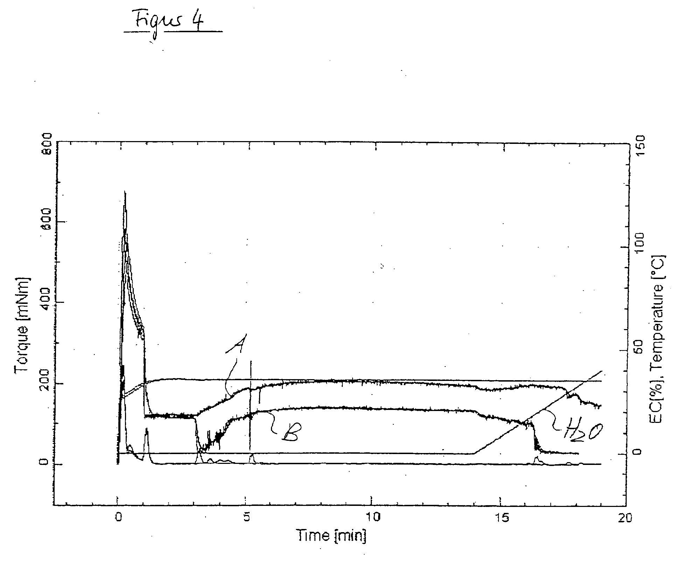

Figur 4 zeigt das Ergebnis einer weiteren Analyse von verwendeten Druckfarben einer in Münster installierten Druckmaschine, wobei gesehen werden kann, dass die Farbe A das Feuchtmittel zu schnell aufnimmt und demzufolge zu mangelhaften Druckergebnissen führt, wohingegen die Farbe B für den Offset-Druck gut geeignet ist.

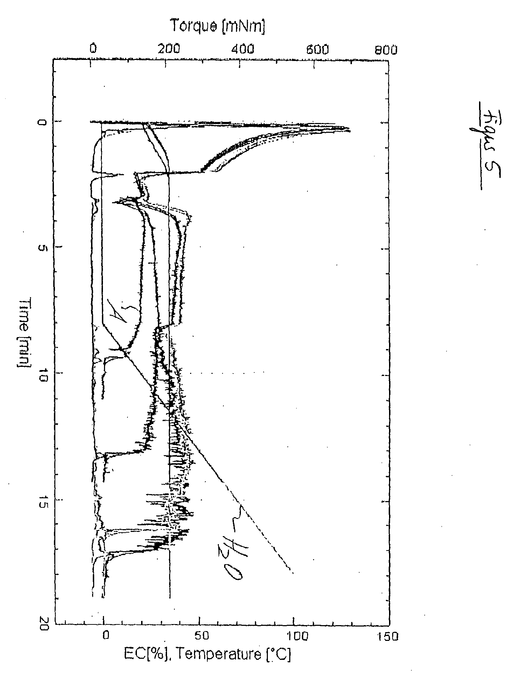

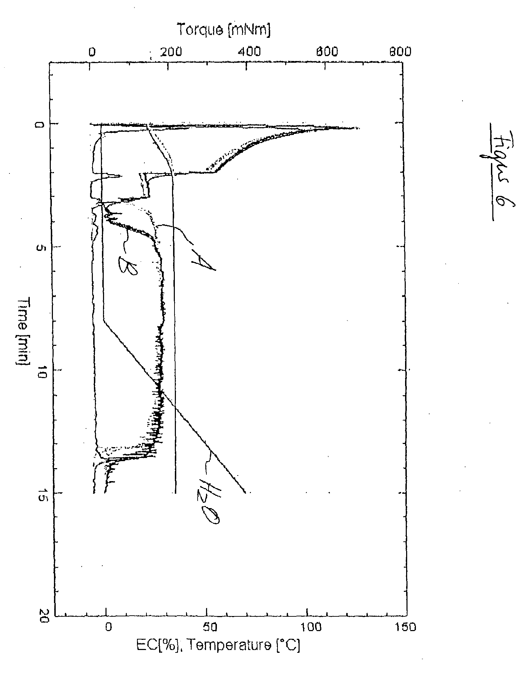

Figuren 5 und 6 zeigen Analyseergebnisse verschiedener Farbsätze bei einer Druckmaschine, wobei insbesondere die mit A gekennzeichnete Farbe zu Problemen führte. Diese Farbe weist eine sehr tiefe Sättigungsgrenze auf, was insbesondere bei hohen Maschinengeschwindigkeiten von über 30000 Zylindern pro Stunde zu Problemen führte. Schon bei einem Feuchtmittelgehalt von 30 % brach das Drehmoment der Farbe A zusammen, was sich als zu niedrig für den Offset-Druck herausgestellt hat. Es wurde empirisch ermittelt, dass die Sättigungsgrenze einer Farbe über 30 % Feuchtmittel liegen sollte, um zufriedenstellende Druckergebnisse zu erzielen. Figur 6 zeigt die Untersuchung von zwei an der gleichen Maschine verwendeten Farben, wobei die Farbe A zu schnell Feuchtmittel aufnimmt und zu schlechten Druckresultaten führte. Bei der untersuchten Farbe B brach nach Feuchtmittelzugabe das Drehmoment für eine kurze Zeit von etwa einer Minute zusammen und stieg danach wieder an. Diese Farbe lieferte gute Druckresultate.

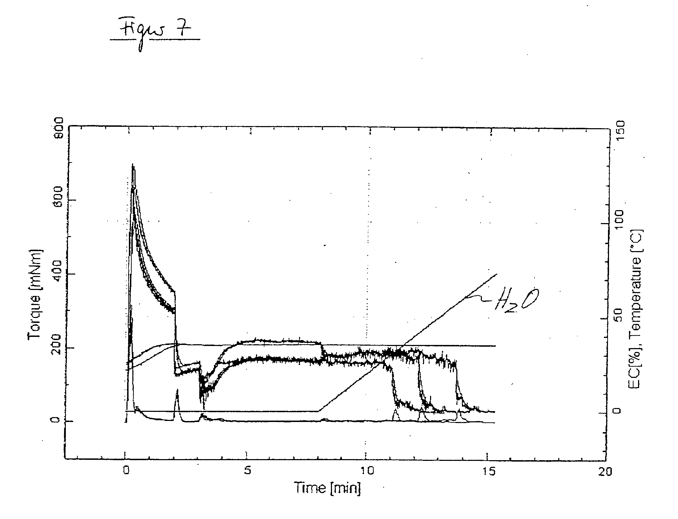

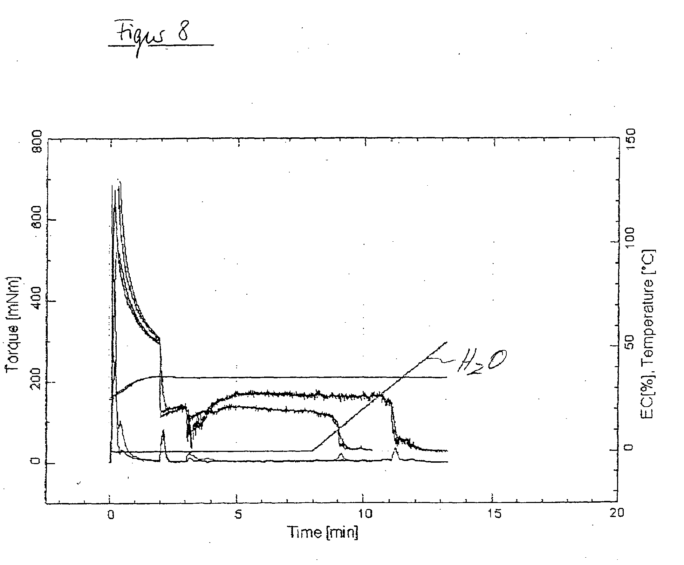

Figuren 7 und 8 zeigen die Analysen zweier bei einem Kurzfarbwerk verwendeten Farbsätze ein- und desselben Lieferanten, wodurch nachgewiesen werden konnte, dass die Qualität der vom Lieferanten gelieferten Farbe -stark schwankte und somit ein Fehlverhalten der Druckmaschine durch qualitativ minderwertige Farben verursacht wurde.

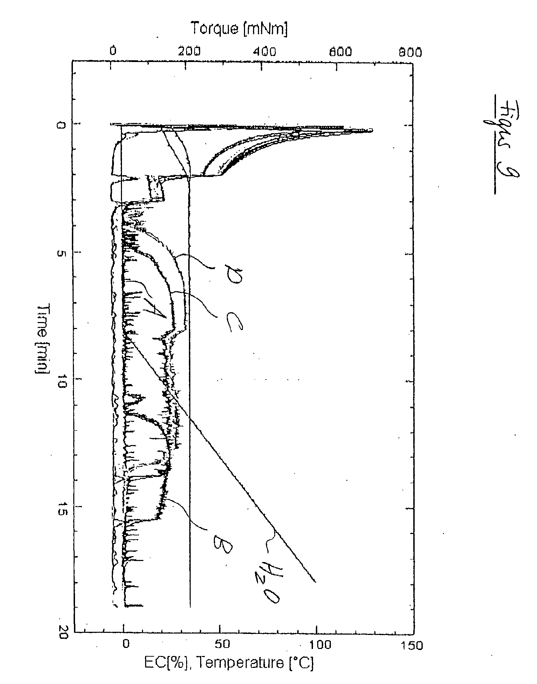

Figur 9 zeigt eine Voruntersuchung eines Farbsatzes für eine Druckmaschine mit Spaltfarbwerk, wobei bereits bei der Voruntersuchung ermittelt werden konnte, dass die untersuchten Farben A und B aufgrund des zu langen Drehmomenteinbruchs und damit zu schlechter Feuchtmittelaufnahmefähigkeit zu Problemen führen werden, während die Farben C und D problemlos verwendbar sein sollten.

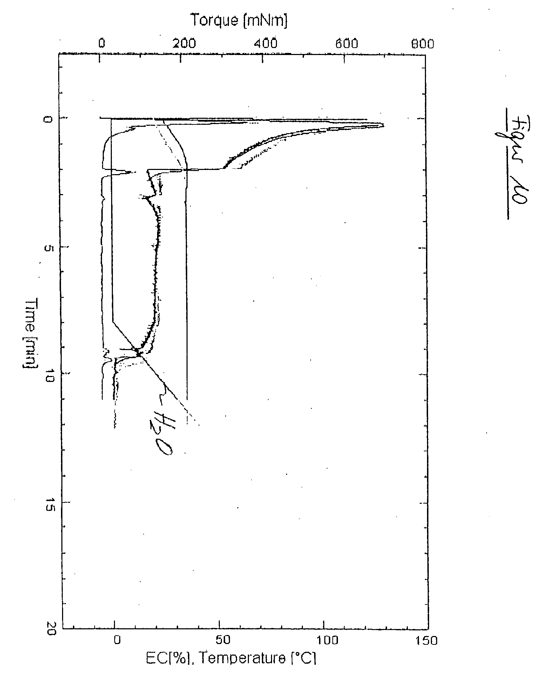

Figur 10 zeigt die Farbanalyse bei aufgetretenem Nebeln und schlechter Förderung im Farbkasten. Es konnte ermittelt werden, dass bei den untersuchten Farben die Sättigungsgrenze zu tief liegt, da unmittelbar nach der kontinuierlichen Wasserzufuhr das Drehmoment zusammenbricht, und erhöht werden muss, um einen qualitativ hochwertigen Druck zu erhalten.

## Patentansprüche

1. Verfahren zur Beurteilung der Qualität einer Druckfarbe, wobei:

   a) eine bestimmte Menge der Druckfarbe gerührt wird;
   b) eine beim Rühren aufzuwendende Kraft oder Energie oder ein aufzuwendendes Moment gemessen wird; und
   c) eine definierte Menge Feuchtmittel zu einem bestimmten Zeitpunkt, bevorzugt auf einmal zur Farbe zugegeben wird.

2. Verfahren nach Anspruch 1, wobei das Rühren durch Drehen eines Drehelements und/oder Verschieben eines Rührelements und/oder Drehen und/oder Kippen eines Farbbehälters durchgeführt wird.

3. Verfahren nach Anspruch 1 oder 2, wobei die Farbe kontinuierlich gerührt wird.

4. Verfahren nach Anspruch 1 oder 2, wobei zuerst mit einer ersten Rate und dann mit einer zweiten Rate gerührt wird, wobei die zweite Rate insbesondere kleiner Rate als die erste Rate ist.

5. Verfahren nach einem der vorhergehenden Ansprüche, wobei die Temperatur der Farbe eingestellt wird.

6. Verfahren nach einem der vorhergehenden Ansprüche, wobei das Feuchtmittel erst zugegeben wird, wenn die beim Rühren aufzuwendende Kraft oder das aufzuwendende Moment in etwa konstant ist.

7. Verfahren nach einem der vorhergehenden Ansprüche, wobei nach einem vorgegebenen Zeitraum nach der Zugabe der definierten Menge des Feuchtmittels kontinuierlich Feuchtmittel bei einer vorgegebenen Rate zugeführt wird.

8. Verfahren zur Bestimmung des Feuchtmittelgehalts einer Farbprobe, wobei:

   a) die Sättigungsgrenze einer Frischfarbe bestimmt wird;
   b) zu der Farbprobe bei andauerndem Rühren Feuchtmittel zugegeben wird;
   c) gemessen wird, wann die zum Rühren aufzuwendende Kraft bzw. das aufzuwendende Drehmoment unter eine vorgegebene Grenze abfällt, und
   d) der Feuchtmittelgehalt aus der ermittelten Sättigungsgrenze der Frischfarbe und dem bei Schritt c) ermittelten Zeitraum bestimmt wird.

9. Vorrichtung zur Beurteilung der Qualität einer Druckfarbe mit:

a) einem Behälter für die Druckfarbe;

b) einer Rührvorrichtung, mit welcher eine in dem Behälter eingefüllte Farbe gerührt werden kann;

c) einer Messvorrichtung zur Messung der von der Rührvorrichtung aufzubringenden Kraft oder dem aufzubringenden Moment;

d) einer Feuchtmittelzufuhr; und

e) einer Steuerung, welche die Feuchtmittelzufuhr so ansteuert, dass eine vorgegebene Menge des Feuchtmittels zu einem definierten Zeitpunkt in den Behälter eingegeben wird.

10. Vorrichtung nach Anspruch 9, wobei eine Temperatureinstellvorrichtung an dem Behälter für die Farbe vorgesehen ist.

11. Vorrichtung nach Anspruch 9 oder 10, wobei die Steuerung zur Durchführung eines oder mehrerer Verfahrensschritte nach einem der Ansprüche 1 bis 8 geeignet ist.

12. Verfahren zur automatischen Farbaufbereitung, wobei eine Farbe mit einem Verfahren nach einem der Ansprüche 1 bis 8 analysiert wird und farbverbessernde Zusätze in Abhängigkeit von dem Analyseergebnis zugeführt werden.

13. Vorrichtung zur automatischen Farbaufbereitung mit einer Vorrichtung nach einem der Ansprüche 9 bis 11, einer Speichervorrichtung für Farbzusätze, welche die Feuchtmittelaufnahmefähigkeit der Farbe verändern, und einer Steuerung, welche die Farbzusätze in Abhängigkeit von dem Analyseergebnis der Farbe zugibt.

Figur 1

Figur 2

Figur 3

Figur 4

Figur 5

Figur 6

Figur 7

Figur 8

Figur 9

Figur 10

Figur 11

Figur 12

Figur 13